# EUROPÄISCHE PATENTANMELDUNG

(11) **EP 3 272 336 A1**
(43) Veröffentlichungstag der Anmeldung: **24.01.2018**
(21) Anmeldenummer: 16180093.3
(22) Anmeldetag: 19.07.2016
(51) Int. Cl.: A61K 9/20, A61K 31/00

(54) **ATORVASTATIN-ZUSAMMENSETZUNG**

(71) Anmelder: STADA ARZNEIMITTEL AG, 61118 Bad Vilbel (DE)
(72) Erfinder: SCHÖNBORN, Jessica, 61118 Bad Vilbel (DE)
(74) Vertreter: Kernebeck, Thomas

(57) **Zusammenfassung**

Die vorliegende Erfindung betrifft eine feste pharmazeutische orale Zusammensetzung, umfassend als Wirkstoff ein Salz des Atorvastatins in kristalliner Form, wobei der Wirkstoff eine Partikelgrößenverteilung mit einem D90-Wert von kleiner als 100 *µ*m aufweist. Um eine solche Zusammensetzung derart weiterzuentwickeln, dass sie eine verhältnismäßig hohe chemische Stabilität bezüglich des Wirkstoffs aufweist, wird vorgeschlagen, dass die Zusammensetzung direktverpresst ist.

## Beschreibung

Die vorliegende Erfindung betrifft eine feste pharmazeutische orale Zusammensetzung, umfassend als Wirkstoff ein Salz des Atorvastatins in kristalliner Form, wobei der Wirkstoff eine Partikelgrößenverteilung mit einem D90-Wert von kleiner als 100 *µ*m aufweist.

Atorvastatin ist der internationale Freiname (INN (International Nonproprietary Name)) für (3R,5R)-7-[2-(4-Fluorphenyl)-5-isopropyl- 3-phenyl-4-(phenylcarbamoyl)pyrrol-1-yl]- 3,5-dihydroxyheptansäure. Atorvastatin weist die folgende Strukturformel auf:

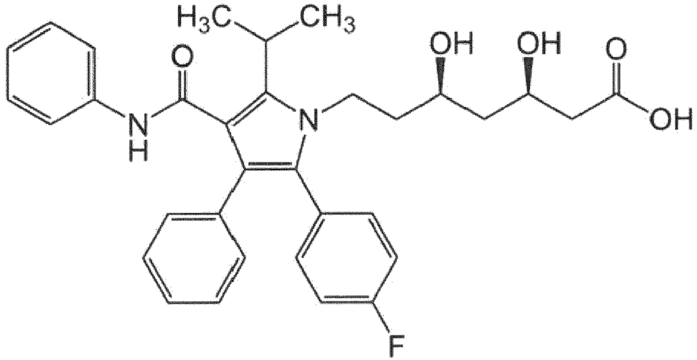

Atorvastatin ist ein Arzneistoff aus der Gruppe der Statine und wird zur Therapie der Hypercholesterinämie eingesetzt. Der Wirkungsmechanismus von Atorvastatin beruht auf einer kompetitiven Hemmung der HMG-CoA-Reduktase, dem Schlüsselenzym der Cholesterolbiosynthese. Die HMG-CoA-Reduktase wirkt als Katalysator bei der Reduktion des 3-Hydroxy-3-methylglutaryl-Coenzym A (HMG-CoA) zu Mevalonat. Durch die Absenkung der Cholesterinsynthese steigern die Leberzellen die Anzahl der LDL-Rezeptoren auf der Zelloberfläche, so dass die LDL-Aufnahme in die Leberzelle erhöht und damit der LDL-Spiegel im Blut verringert wird (vgl. Mutschler Arzneimittelwirkungen, Lehrbuch der Pharmakologie und Toxikologie, 9. Auflage, Wissenschaftliche Verlagsgesellschaft mbH Stuttgart, 2008, S. 534 ff.; ISBN 978-3-8047-1952-1).

Atorvastatin weist eine verhältnismäßig schlechte Löslichkeit in wässrigen Medien auf und wird in die Klasse II des biopharmazeutischen Klassifizierungssystems (englisch: Biopharmaceutics Classification System (BCS)) eingeordnet. Klasse II-Wirkstoffe sind durch eine vergleichsweise niedrige Löslichkeit verbunden mit einem vergleichsweise hohen Permeationsvermögen gekennzeichnet, weshalb bei Klasse II-Wirkstoffen die Resorption durch die Löslichkeit und/oder die Lösungsgeschwindigkeit des Wirkstoffes kontrolliert wird.

Aufgrund seiner relativ schlechten Löslichkeit wird Atorvastatin in festen pharmazeutischen oralen Zusammensetzungen in der Regel in Form eines seiner Salze eingesetzt. Da aber auch Atorvastatinsalze eine vergleichsweise niedrige Löslichkeit und Lösungsgeschwindigkeit aufweisen, werden die Salze entweder in amorpher Form oder mikronisierter kristalliner Form in die Zusammensetzungen eingearbeitet. Da amorphe Atorvastatinsalze allerding eine relativ starke Kristallisationstendenz zeigen und damit zu einer Veränderung ihrer Löslichkeitscharakteristik neigen, werden mikronisierte kristalline Atorvastatinsalze in der Präparation von festen pharmazeutischen oralen Formulierungen im Allgemeinen bevorzugt.

Insbesondere in mikronisierter Form weisen Atorvastatin und seine Salze eine vergleichsweise geringe Stabilität auf (vgl. auch WO 2005/011638).

Aufgabe der vorliegenden Erfindung ist es daher, eine feste pharmazeutische orale Zusammensetzung der eingangs genannten Art bereitzustellen, die durch eine verhältnismäßig hohe Stabilität des Wirkstoffs gekennzeichnet ist.

Unter "Stabilität" wird in diesem Zusammenhang die von der Arbeitsgemeinschaft für pharmazeutische Verfahrenstechnik (APV) erarbeitete Definition verstanden, nach welcher "Stabilität" die spezifikationsgerechte Qualität des Arzneimittels bis zum Ende der vom Hersteller festgelegten Laufzeit bedeutet. Die Qualität des Arzneimittels wird dabei durch den Wirkstoffgehalt und die Reinheit, die sensorisch wahrnehmbaren, physikalisch-chemischen und die mikrobiologischen Eigenschaften bestimmt, wobei der Wirkstoffgehalt bis zum Ende der Laufzeit 90 % des deklarierten Wertes nicht unterschreiten soll.

Überraschenderweise wurde aufgefunden, dass eine feste pharmazeutische orale Zusammensetzung, umfassend als Wirkstoff ein Salz des Atorvastatins in kristalliner Form, wobei der Wirkstoff eine Partikelgrößenverteilung mit einem D90-Wert von kleiner als 100 *µ*m aufweist, eine verhältnismäßig hohe Stabilität bezüglich des Wirkstoffs aufweist, wenn die Zusammensetzung direktverpresst ist.

Direktverpresste Zusammensetzungen - oder mit Bezug auf Tabletten: "direkttablettierte Zusammensetzungen" - und Verfahren zu deren Herstellung sind im Stand der Technik bekannt, z.B. aus "Die Tablette", A. Bauer-Brandl et al., 3. Auflage, Editio-Cantor-Verlag, 2012, ISBN 978-3-87193-407-0.

Darüber hinaus hat die erfindungsgemäße Zusammensetzung den Vorteil, dass sie verhältnismäßig kostengünstig ist. Da die erfindungsgemäße Zusammensetzung direktverpresst ist, entfallen bei Ihrer Herstellung beispielsweise Schritte, die eine Granulierung des Wirkstoffs und den Einsatz von Lösungsmitteln z.B. als Granulierflüssigkeit vorsehen. Daher ist die erfindungsgemäße Zusammensetzung verhältnismäßig kostengünstig herstellbar.

Ferner wurde festgestellt, dass die pulverförmige Mischung zur Herstellung der erfindungsgemäßen Zusammensetzung - obwohl sie den Wirkstoff in frei fließender mikronisierter Form enthält - eine ausreichende Fließfähigkeit aufweisen kann, um auf einer konventionellen industriellen Pressvorrichtung relativ schnell in großer Stückzahl zu Komprimaten verarbeitet zu werden. Dadurch ist ebenfalls gewährleistet, dass die erfindungsgemäße Zusammensetzung vergleichsweise kostengünstig hergestellt werden kann.

Gemäß einer bevorzugten Ausführungsform der erfindungsgemäßen Zusammensetzung ist es vorgesehen, dass die Zusammensetzung ferner einen Hydrogelbildner umfasst. Es konnte festgestellt werden, dass die Wirkstofffreisetzung der erfindungsgemäßen Zusammensetzung besonders wirksam mittels eines Hydrogelbildners variiert werden kann. Hydrogele und Hydrogelbildner sind im Stand der Technik bekannt, z.B. aus "Die Tablette", A. Bauer-Brandl et al., 3. Auflage, Editio-Cantor-Verlag, 2012, ISBN 978-3-87193-407-0 oder aus Schöffling, Arzneiformenlehre Ein Lehrbuch der Galenik für Theorie und Praxis, 5. Auflage, Deutscher Apotheker Verlag, 2009, ISBN 978-3-7692-4093-1.

Entsprechend einer weiter bevorzugten Ausführungsform der erfindungsgemäßen Zusammensetzung ist es vorgesehen, dass der Wirkstoff und der Hydrogelbildner im Gemisch miteinander vorliegen. Durch diese Maßnahme kann die gewünschte Wirkstofffreisetzung der erfindungsgemäßen Zusammensetzung besonders effektiv und einfach eingestellt werden.

Nach einer weiter bevorzugten Ausführungsform der erfindungsgemäßen Zusammensetzung ist es vorgesehen, dass der Hydrogelbildner in der Zusammensetzung mit einem Anteil von 0,1 Gew.-% bis 30 Gew.-% enthalten ist bezogen auf das Gesamtgewicht der Zusammensetzung, mehr bevorzugt mit einem Anteil von 0,5 Gew.-% bis 10 Gew.-% und weiter bevorzugt mit einem Anteil von 1,0 Gew.-% bis 4 Gew.-%. In den genannten Anteilsintervallen kann die gewünschte Wirkstofffreisetzung der erfindungsgemäßen Zusammensetzung besonders effektiv und einfach sichergestellt werden.

In einer weiter bevorzugten Ausführungsform der erfindungsgemäßen Zusammensetzung ist es vorgesehen, dass der Hydrogelbildner ausgewählt ist aus der Gruppe bestehend aus organischen und anorganischen Hydrogelbildnern. Es konnte festgestellt werden, dass sowohl organische als auch anorganische Hydrogelbildner geeignet sind, um die gewünschte Wirkstofffreisetzung der erfindungsgemäßen Zusammensetzung sicherzustellen.

Gemäß einer weiter bevorzugten Ausführungsform der erfindungsgemäßen Zusammensetzung ist es vorgesehen, dass der organische Hydrogelbildner ausgewählt ist aus der Gruppe bestehend aus Agar, Alginsäure und Alginat, arabisches Gummi, Gelatine, Tragant, Kartoffelstärke, Maisstärke, Reisstärke, Weizenstärke, Polyacrylat, Polyvinylalkohol, Polyvinylpyrrolidon, Copolyvidon und Celluloseether. Die genannten Hydrogelbildner haben sich als pharmazeutisch anerkannte Hilfsstoffe auch aus verarbeitungstechnischer Sicht als besonders geeignet für die erfindungsgemäße Zusammensetzung erwiesen.

Entsprechend einer weiter bevorzugten Ausführungsform der erfindungsgemäßen Zusammensetzung ist es vorgesehen, dass der Celluloseether ausgewählt ist aus der Gruppe bestehend aus Methylcellulose, Methylhydroxyethylcellulose, Hydroxypropylmethylcellulose, Hydroxyethylcellulose, Hydroxypropylcellulose und Natriumcarboxymethylcellulose, mehr bevorzugt aus der Gruppe bestehend aus Hydroxypropylmethylcellulose. Mittels der genannten, auf Cellulose basierenden Hydrogelbildner kann die Wirkstofffreisetzung der erfindungsgemäßen Zusammensetzung besonders effektiv und einfach sichergestellt werden.

Nach einer weiter bevorzugten Ausführungsform der erfindungsgemäßen Zusammensetzung ist es vorgesehen, dass der anorganische Hydrogelbildner ausgewählt ist aus der Gruppe bestehend aus Bentonit, Siliziumdioxid und Aluminium-Magnesium-Silikat. Mittels der genannten anorganischen Hydrogelbildner kann die Wirkstofffreisetzung der erfindungsgemäßen Zusammensetzung zufriedenstellend sichergestellt werden.

In einer weiter bevorzugten Ausführungsform der erfindungsgemäßen Zusammensetzung ist es vorgesehen, dass der Hydrogelbildner eine dynamische Viskosität in einem Bereich von 50 cP bis 500 cP aufweist, mehr bevorzugt eine dynamische Viskosität in einem Bereich von 50 cP bis 200 cP. Mittels Hydrogelbildner besagter Spezifikation kann die Wirkstofffreisetzung der erfindungsgemäßen Zusammensetzung besonders effektiv und einfach sichergestellt werden.

Die dynamische Viskosität ist gemäß dem Europäischen Arzneibuch (Ph.Eur.), 8. Ausgabe, Grundwerk 2014, S. 35-36, Kapitel 2.2. 9 "Kapillarviskosimeter" mit einer 2 %igen Mischung des Gelbildners in Wasser bei einer Temperatur von 20 ± 0,1 °C zu bestimmen.

In der erfindungsgemäßen Zusammensetzung ist als Wirkstoff ein kristallines Atorvastatinsalz in mikronisierter Form enthalten, d.h., dass der Wirkstoff eine Partikelgrößenverteilung mit einem D90-Wert von kleiner als 100 *µ*m aufweist. Gemessen wird der D90-Wert mittels des Wirkstoffs in freier pulvriger Form.

Gemäß einer weiter bevorzugten Ausführungsform der erfindungsgemäßen Zusammensetzung ist es vorgesehen, dass der Wirkstoff eine Partikelgrößenverteilung mit einem D90-Wert von kleiner als 20 *µ*m aufweist. Auch im Hinblick auf eine Partikelgrößenverteilung mit besonders kleinem D90-Wert zeigt die erfindungsgemäße Zusammensetzung eine verhältnismäßig hohe Stabilität bzgl. des Wirkstoffs.

Entsprechend einer weiter bevorzugten Ausführungsform der erfindungsgemäßen Zusammensetzung ist es vorgesehen, dass der Wirkstoff eine Partikelgrößenverteilung mit einem D10-Wert von 0,6 +/- 0,5 *µ*m, einem D50-Wert von 1,8 +/- 0,5 *µ*m und einem D90-Wert von 5,5 +/- 0,5 *µ*m aufweist.

Unter D10-Wert wird in diesem Zusammenhang die Partikelgröße verstanden, bei der 10 % der Partikel bezogen auf das Volumen kleiner als der D10-Wert sind und 90 % der Partikel bezogen auf das Volumen größer sind als der D10-Wert. In Analogie dazu wird unter D50-Wert in vorgenanntem Zusammenhang die Partikelgröße verstanden, bei der 50 % der Partikel bezogen auf das Volumen kleiner als der D50-Wert sind und 50 % der Partikel bezogen auf das Volumen größer sind als der D50-Wert. In Analogie dazu wird unter D90-Wert in vorgenannten Zusammenhängen die Partikelgröße verstanden, bei der 90 % der Partikel bezogen auf das Volumen kleiner als der D90-Wert sind und 10 % der Partikel bezogen auf das Volumen größer sind als der D90-Wert.

Der D10-, der D50- und der D90-Wert des Wirkstoffs ist gemäß Ph. Eur., 8. Ausgabe, Kapitel 2.9.31 "Bestimmung der Partikelgröße durch Laserdiffraktometrie", Seiten 455-460 zu bestimmen. Dabei wird erfindungsgemäß bevorzugt der D10-, der D50- und der D90-Wert mittels Laserbeugung unter Einsatz eines Gerätes mit der Bezeichnung "Mastersizer 2000" der Firma "Malvern Instruments" und dem Datenanalyseprogramm Malvern Application v5.60 ermittelt. Die Geräte-/Programmeinstellungen sind dabei wie folgt: Dispergiersystem (dispersing system) Scirocco 2000; Messbereich (result range) 0,02 bis 2000 *µ*m; Auswertung (reporting) Fraunhofer; Berechnungsmodel (calculation model) Fraunhofer; Berechnungsempfindlichkeit (calculation sensitivity) verstärkt (enhanced); Messzeit (measurement time) 15 s; Hintergrundzeit (background time) 20 s; Messaufnahmen (measurement snaps) 15.000; Hintergrundaufnahmen (background snaps) 20.000; Dispergiermethode (dispersing method) Trockenmessung, das Pulver wird in Luft dispergiert; Einsatz (tray) universell (general purpose), 6 mm Öffnung; Probennehmereinstellungen (sampler settings) Vibrationszuführrate (vibration feed rate) 25 %, Luftdruck zur Dispergierung (dispersive air pressure) 0,5 bar, Sieb: grob; Trübungsgrenzen (obscuration limits) Untergrenze 0,5 %, Obergrenze 6 %; Anzahl der Messungen 3.

Nach einer weiter bevorzugten Ausführungsform der erfindungsgemäßen Zusammensetzung ist es vorgesehen, dass der Wirkstoff ein Kalziumsalz, ein Magnesiumsalz, ein Aluminiumsalz, ein Eisensalz oder ein Zinksalz des Atorvastatins ist.

In einer weiter bevorzugten Ausführungsform der erfindungsgemäßen Zusammensetzung ist es vorgesehen, dass der Wirkstoff ein Hemikalziumsalz des Atorvastatins ist.

Gemäß einer weiter bevorzugten Ausführungsform der erfindungsgemäßen Zusammensetzung ist es vorgesehen, dass der Wirkstoff ein Trihydrat des Hemikalziumsalzes des Atorvastatins ist.

Entsprechend einer weiter bevorzugten Ausführungsform der erfindungsgemäßen Zusammensetzung ist es vorgesehen, dass der Wirkstoff ein Hemikalziumsalz des Atorvastatins ist, dass ein unter Verwendung von Cu-Kₐₗₚₕₐ-Strahlung (Wellenlänge lambda = 1,5406 Angström, Stromstärke: 20 mA, Spannung: 40 kV) erhaltenes Pulver-Röntgenbeugungsmuster mit Signalen bei den Beugungswinkeln 2thetha von 4,12°, 4,99°, 5,77°, 7,67°, 8,45°, 15,96°, 16,62°, 17,73°, 18,27°, 18,87°, 19,48°, 19,98°, 20,29°, 21,11°, 21,67°, 23,32°, 24,41°, 24,97° und 25,40° ± 0,2° aufweist. Die besagte polymorphe Form des Hemikalziumsalz des Atorvastatins ist unter der Bezeichnung Form III aus EP 0 848 704 bekannt.

Nach einer weiter bevorzugten Ausführungsform der erfindungsgemäßen Zusammensetzung ist es vorgesehen, dass die Zusammensetzung eine Tablette oder eine Kapsel ist. Dabei kann die Tablette beispielsweise nur aus einem Tablettenkern bestehen oder aus einem Tablettenkern, der befilmt ist.

In einer weiter bevorzugten Ausführungsform der erfindungsgemäßen Zusammensetzung ist es vorgesehen, dass die Zusammensetzung frei von einem Tensid ist, insbesondere frei von Polysorbaten, beispielsweise frei von Polysorbat 80.

Gemäß einer weiter bevorzugten Ausführungsform der erfindungsgemäßen Zusammensetzung ist es vorgesehen, dass die Zusammensetzung eine *in vitro*-Freisetzungsrate des Wirkstoffs, gemessen unter Anwendung der Blattrührer-Methode gemäß Eur. Ph. bei 75 U/min in 900 ml Phosphatpuffer mit einem pH-Wert von 6,8 bei 37 ± 0,5 °C, von 35 % bis 50 % in 5 min, von 65 % bis 90 % in 10 min, von 70 % bis 95 % in 15 min, von 80 % bis 100 % in 30 min und von 85 % bis 100 % in 45 min aufweist.

Die in *vitro*-Freisetzungsrate wird unter Anwendung der Blattrührer-Apparatur (Apparatur 2) und der Blattrührer-Methode gemäß Eur.Ph. (Europäisches Arzneibuch, 7. Ausgabe, 3. Nachtrag, 2.9.3 "Wirkstofffreisetzung aus festen Arzneiformen", S. 5519-5526) bei 75 U/min in 900 ml Phosphatpuffer (34,05 g Kaliumdihydrogenphosphat werden in 112 ml 1 molare NaOH gelöst und die Lösung mit dest. Wasser auf ein Volumen von 5 1 aufgefüllt; ggf. wird der pH-Wert auf 6,8 eingestellt) mit einem pH-Wert von 6,8 bei 37 ± 0,5 °C bestimmt. Die Menge an freigesetztem Wirkstoff wird vorzugsweise mittels UV-Detektion bei 246 nm bestimmt.

Entsprechend einer weiter bevorzugten Ausführungsform der erfindungsgemäßen Zusammensetzung ist es vorgesehen, dass der Anteil der Zusammensetzung an Wirkstoff 5 Gew.-% bis 20 Gew.-% beträgt, mehr bevorzugt 5 Gew.-% bis 15 Gew.-%.

Nach einer weiter bevorzugten Ausführungsform der erfindungsgemäßen Zusammensetzung ist es vorgesehen, dass der Anteil der Zusammensetzung an Wirkstoff 0,5 mg bis 100 mg beträgt, mehr bevorzugt 10 mg bis 80 mg.

In einer weiter bevorzugten Ausführungsform der erfindungsgemäßen Zusammensetzung ist es vorgesehen, dass die Zusammensetzung einen oder mehrere pharmazeutische Hilfsstoffe umfasst, ausgewählt aus der Gruppe bestehend aus Füllmitteln, Sprengmitteln, Bindemitteln, Fließregulierungsmitteln, Schmiermitteln und Formentrennmitteln.

Gemäß einer weiter bevorzugten Ausführungsform der erfindungsgemäßen Zusammensetzung ist es vorgesehen, dass die Zusammensetzung ein granuläres Trockenbindemittel umfasst. Dadurch wird eine verbesserte Fließfähigkeit der der erfindungsgemäßen Zusammensetzung zugrundeliegenden pulverförmigen Mischung gewährleistet. Granuläre Trockenbindemittel sind im Stand der Technik bekannt. Dabei handelt es sich um Trockenbindemittel in Granulatform, die vorzugsweise mittels Trocken- oder Feuchtgranulierung hergestellt sind.

Eine typische, erfindungsgemäß bevorzugte feste pharmazeutische orale Zusammensetzung ist beispielsweise eine direktverpresste - oder anders gesagt direkttablettierte - Tablette mit oder ohne Befilmung, umfassend als Wirkstoff ein Hemikalziumsalz des Atorvastatins in kristalliner Form, wobei der Wirkstoff eine Partikelgrößenverteilung mit einem D90-Wert von kleiner als 100 *µ*m aufweist, vorzugsweise eine Partikelgrößenverteilung mit einem D90-Wert von kleiner als 20 *µ*m, wobei die Zusammensetzung ferner einen Hydrogelbildner umfasst, vorzugsweise eine Hydroxypropymethylcellulose, die ebenfalls bevorzugt eine dynamische Viskosität in einem Bereich von 50 cP bis 500 cP aufweist.

Die vorliegende Erfindung betrifft ferner eine pulverförmige Mischung zur Herstellung der erfindungsgemäßen Zusammensetzung, umfassend als Wirkstoff ein Salz des Atorvastatins in kristalliner frei fließender Form, wobei der Wirkstoff eine Partikelgrößenverteilung mit einem D90-Wert von kleiner als 100 *µ*m aufweist.

Die vorliegende Erfindung betrifft ferner die Verwendung der erfindungsgemäßen pulverförmigen Mischung zur Herstellung der erfindungsgemäßen Zusammensetzung.

Die vorliegende Erfindung betrifft ferner ein Verfahren zur Herstellung der erfindungsgemäßen Zusammensetzung, umfassend den Schritt des Verpressens der erfindungsgemäßen pulverförmigen Mischung.

Die nachfolgenden Ausführungsbeispiele dienen der Erläuterung der Erfindung.

### Ausführungsbeispiel 1:

Es wurden Filmtabletten als erfindungsgemäße Zusammensetzung mit der in Tabelle 1 angegebenen Formulierung hergestellt:

**Tabelle 1:**

| **Substanz** | **Menge pro Tablette [mg]** | **Funktion der Substanz** |
|---|---|---|
| **Tablettenkern** | | |
| Atorvastatin-Hemikalzium-Trihydrat (kristalline Form III, vgl. EP 0 848 704; D10 = 0,610 *µ*m, D50 = 1,832 *µ*m, D90 = 5,465 *µ*m) | 86,59 | Wirkstoff |
| Cellactose^{®} (sprühgetrocknete Mischung von 75 Gew.-% alpha-Lactose-Monohydrat und 25 Gew.-% Pulvercellulose) | 432,26 | Füll- und Trockenbindemit tel |
| Destab^{®} (Granulat aus 90 Gew.-% CaCO₃ und 10 Gew.-% vorgelatinierte Stärke) | 227,00 | Füll- und Trockenbindemit tel |
| Croscarmellose-Natrium | 48,00 | Sprengmittel |
| Magnesiumstearat | 14,15 | Schmiermittel |

| **Film** | | |
|---|---|---|
| Opadry^{®} weiss (enthält Hypromellose, Polyethylenglycol (Macrogol 8000), Talkum, Titandioxid) | 16,00 | vollständiges Befilmungssystem |
| **Gesamtgewicht der Filmtablette** | 824,00 | |

Die Filmtabletten wurden wie folgt hergestellt: Atorvastatin Hemikalzium, Cellactose^{®}, Destab^{®}, Croscarmellose-Natrium und Magnesiumstearat wurden in einem konventionellen Mischer bis zur Homogenität miteinander vermischt unter Erhalt der erfindungsgemäßen pulverförmigen Mischung. Diese pulverförmige Mischung wurde mittels einer konventionellen Tablettenpresse gemäß dem erfindungsgemäßen Verfahren zu Tablettenkernen direktverpresst.

Die so erhaltenen Tablettenkerne wurden in einer konventionellen Filmbeschichtungsvorrichtung durch Besprühen mit einer Beschichtungssuspension befilmt. Die Beschichtungssuspension wurde hergestellt durch Suspendieren des kommerziell erhältlichen Befilmungssystems Opadry® weiss in Wasser.

Die resultierenden Filmtabletten weisen eine in *vitro-*Freisetzungsrate des Wirkstoffs, gemessen unter Anwendung der Blattrührer-Methode gemäß Eur. Ph. bei 75 U/min in 900 ml Phosphatpuffer mit einem pH-Wert von 6,8 bei 37 ± 0,5 °C, von 78 % in 5 min, von 87 % in 10 min, von 90 % in 15 min, von 95 % in 30 min und von 97 % in 45 min auf.

### Ausführungsbeispiel 2:

Es wurden Filmtabletten als erfindungsgemäße Zusammensetzung mit der in Tabelle 2 angegebenen Formulierung hergestellt:

**Tabelle 2:**

| **Substanz** | **Menge pro Tablette [mg]** | **Funktion der Substanz** |
|---|---|---|
| **Tablettenkern** | | |
| Atorvastatin-Hemikalzium-Trihydrat (kristalline Form III, vgl. EP 0 848 704; D10 = 0,610 *µ*m, D50 = 1,832 *µ*m, D90 = 5,465 *µ*m) | 86,59 | Wirkstoff |
| Cellactose^{®} (sprühgetrocknete Mischung von 75 Gew.-% alpha-Lactose-Monohydrat und 25 Gew.-% Pulvercellulose) | 416,10 | Füll- und Trockenbindemit tel |
| Destab^{®} (Granulat aus 90 Gew.-% CaCO₃ und 10 Gew.-% vorgelatinierte Stärke) | 227,00 | Füll- und Trockenbindemit tel |
| Croscarmellose-Natrium | 48,00 | Sprengmittel |
| Hydroxypropylmethylcellulose (HPMC, dynamische Viskosität: 100 cp) | 16,16 | Hydrogelbildner |
| Magnesiumstearat | 14,15 | Schmiermittel |

| **Film** | | |
|---|---|---|
| Opadry^{®} weiss (enthält Hypromellose, Polyethylenglycol (Macrogol 8000), Talkum, Titandioxid) | 16,00 | vollständiges Befilmungssystem |
| **Gesamtgewicht der Filmtablette** | 824,00 | |

Die Filmtabletten wurden wie folgt hergestellt: Atorvastatin Hemikalzium, Cellactose^{®}, Destab^{®}, Croscarmellose-Natrium, HPMC und Magnesiumstearat wurden in einem konventionellen Mischer bis zur Homogenität miteinander vermischt unter Erhalt der erfindungsgemäßen pulverförmigen Mischung. Diese pulverförmige Mischung wurde mittels einer konventionellen Tablettenpresse gemäß dem erfindungsgemäßen Verfahren zu Tablettenkernen direktverpresst.

Die so erhaltenen Tablettenkerne wurden in einer konventionellen Filmbeschichtungsvorrichtung durch Besprühen mit einer Beschichtungssuspension befilmt. Die Beschichtungssuspension wurde hergestellt durch Suspendieren des kommerziell erhältlichen Befilmungssystems Opadry^{®} weiss in Wasser.

Die resultierenden Filmtabletten weisen eine *in vitro-*Freisetzungsrate des Wirkstoffs, gemessen unter Anwendung der Blattrührer-Methode gemäß Eur. Ph. bei 75 U/min in 900 ml Phosphatpuffer mit einem pH-Wert von 6,8 bei 37 ± 0,5 °C, von 43 % in 5 min, von 71 % in 10 min, von 78 % in 15 min, von 88 % in 30 min und von 94 % in 45 min auf.

### Ausführungsbeispiel 3:

Es wurden Filmtabletten als erfindungsgemäße Zusammensetzung mit der in Tabelle 3 angegebenen Formulierung hergestellt:

**Tabelle 3:**

| **Substanz** | **Menge pro Tablette [mg]** | **Funktion der Substanz** |
|---|---|---|
| **Tablettenkern** | | |
| Atorvastatin-Hemikalzium-Trihydrat (kristalline Form III, vgl. EP 0 848 704; D10 = 0,610 *µ*m, D50 = 1,832 *µ*m, D90 = 5,465 *µ*m) | 86,59 | Wirkstoff |
| Cellactose^{®} (sprühgetrocknete Mischung von 75 Gew.-% alpha-Lactose-Monohydrat und 25 Gew.-% Pulvercellulose) | 408,02 | Füll- und Trockenbindemit tel |
| Destab^{®} (Granulat aus 90 Gew.-CaCO₃ und 10 Gew.-% vorgelatinierte Stärke) | 227,00 | Füll- und Trockenbindemit tel |
| Croscarmellose-Natrium | 48,00 | Sprengmittel |
| Hydroxypropylmethylcellulose (HPMC, dynamische Viskosität: 100 cp) | 24,24 | Hydrogelbildner |
| Magnesiumstearat | 14,15 | Schmiermittel |

| **Film** | | |
|---|---|---|
| Opadry^{®} weiss (enthält Hypromellose, Polyethylenglycol (Macrogol 8000), Talkum, Titandioxid) | 16,00 | vollständiges Befilmungssystem |
| **Gesamtgewicht der Filmtablette** | 824,00 | |

Die Filmtabletten wurden analog Ausführungsbeispiel 2 hergestellt.

Die resultierenden Filmtabletten weisen eine in *vitro-*Freisetzungsrate des Wirkstoffs, gemessen unter Anwendung der Blattrührer-Methode gemäß Eur. Ph. bei 75 U/min in 900 ml Phosphatpuffer mit einem pH-Wert von 6,8 bei 37 ± 0,5 °C, von 23 % in 5 min, von 60 % in 10 min, von 74 % in 15 min, von 85 % in 30 min und von 90 % in 45 min auf.

### Ausführungsbeispiel 4:

Es wurden Filmtabletten als erfindungsgemäße Zusammensetzung mit der in Tabelle 4 angegebenen Formulierung hergestellt:

**Tabelle 4:**

| **Substanz** | **Menge pro Tablette [mg]** | **Funktion der Substanz** |
|---|---|---|
| **Tablettenkern** | | |
| | | |
| Atorvastatin-Hemikalzium-Trihydrat (kristalline Form III, vgl. EP 0 848 704; D10 = 0,610 *µ*m, D50 = 1,832 *µ*m, D90 = 5,465 *µ*m) | 86,59 | Wirkstoffs |
| Cellactose^{®} (sprühgetrocknete Mischung von 75 Gew.-% alpha-Lactose-Monohydrat und 25 Gew.-% Pulvercellulose) | 399,94 | Füll- und Trockenbindemit tel |
| Destab^{®} (Granulat aus 90 Gew.-% CaCO₃ und 10 Gew.-% vorgelatinierte Stärke) | 227,00 | Füll- und Trockenbindemit tel |
| Croscarmellose-Natrium | 48,00 | Sprengmittel |
| Hydroxypropylmethylcellulose (HPMC, dynamische Viskosität: 100 cp) | 32,32 | Hydrogelbildner |
| Magnesiumstearat | 14,15 | Schmiermittel |

| **Film** | | |
|---|---|---|
| Opadry^{®} weiss (enthält Hypromellose, Polyethylenglycol (Macrogol 8000), Talkum, Titandioxid) | 16,00 | vollständiges Befilmungssystem |
| **Gesamtgewicht der Filmtablette** | 824,00 | |

Die Filmtabletten wurden analog Ausführungsbeispiel 2 hergestellt.

Die resultierenden Filmtabletten weisen eine *in vitro-*Freisetzungsrate des Wirkstoffs, gemessen unter Anwendung der Blattrührer-Methode gemäß Eur. Ph. bei 75 U/min in 900 ml Phosphatpuffer mit einem pH-Wert von 6,8 bei 37 ± 0,5 °C, von 20 % in 5 min, von 47 % in 10 min, von 64 % in 15 min, von 82 % in 30 min und von 90 % in 45 min auf.

### Ausführungsbeispiel 5:

Es wurden Filmtabletten als erfindungsgemäße Zusammensetzung mit der in Tabelle 5 angegebenen Formulierung hergestellt:

**Tabelle 5:**

| **substanz** | **menge pro Tablette [mg]** | **Funktion der Substanz** |
|---|---|---|
| **Tablettenkern** | | |
| Atorvastatin-Hemikalzium-Trihydrat (kristalline Form III, vgl. EP 0 848 704; D10 = 0,610 *µ*m, D50 = 1,832 *µ*m, D90 = 5,465 *µ*m) | 86,59 | Wirkstoff |
| Cellactose^{®} (sprühgetrocknete Mischung von 75 Gew.-% alpha-Lactose-Monohydrat und 25 Gew.-% Pulvercellulose) | 408,02 | Füll- und Trockenbindemit tel |
| Destab^{®} (Granulat aus 90 Gew.-% CaCO₃ und 10 Gew.-% vorgelatinierte Stärke) | 227,00 | Füll- und Trockenbindemit tel |
| Croscarmellose-Natrium | 48,00 | Sprengmittel |
| Hydroxypropylmethylcellulose (HPMC, dynamische Viskosität: 4000 cp) | 24,24 | Hydrogelbildner |
| Magnesiumstearat | 14,15 | Schmiermittel |

| **Film** | | |
|---|---|---|
| Opadry^{®} weiss (enthält Hypromellose, Polyethylenglycol (Macrogol 8000), Talkum, Titandioxid) | 16,00 | vollständiges Befilmungs-system |
| **Gesamtgewicht der Filmtablette** | 824,00 | |

Die Filmtabletten wurden analog Ausführungsbeispiel 2 hergestellt.

## Patentansprüche

1. Feste pharmazeutische orale Zusammensetzung, umfassend als Wirkstoff ein Salz des Atorvastatins in kristalliner Form, wobei der Wirkstoff eine Partikelgrößenverteilung mit einem D90-Wert von kleiner als 100 *µ*m aufweist, **dadurch gekennzeichnet, dass** die Zusammensetzung direktverpresst ist.

2. Zusammensetzung nach Anspruch 1, **dadurch gekennzeichnet, dass** die Zusammensetzung ferner einen Hydrogelbildner umfasst.

3. Zusammensetzung nach Anspruch 2, **dadurch gekennzeichnet, dass** der Wirkstoff und der Hydrogelbildner im Gemisch miteinander vorliegen.

4. Zusammensetzung nach Anspruch 2 oder 3, **dadurch gekennzeichnet, dass** der Hydrogelbildner eine dynamische Viskosität in einem Bereich von 50 cP bis 500 cP aufweist, mehr bevorzugt eine dynamische Viskosität in einem Bereich von 50 cP bis 200 cP.

5. Zusammensetzung nach einem der voranstehenden Ansprüche, **dadurch gekennzeichnet, dass** der Wirkstoff eine Partikelgrößenverteilung mit einem D90-Wert von kleiner als 20 *µ*m aufweist.

6. Zusammensetzung nach einem der voranstehenden Ansprüche, **dadurch gekennzeichnet, dass** der Wirkstoff eine Partikelgrößenverteilung mit einem D10-Wert von 0,6 +/- 0,5 *µ*m, einem D50-Wert von 1,8 +/- 0,5 *µ*m und einem D90-Wert von 5,5 5 +/- 0,5 *µ*m aufweist.

7. Zusammensetzung nach einem der voranstehenden Ansprüche, **dadurch gekennzeichnet, dass** der Wirkstoff ein Hemikalziumsalz des Atorvastatins ist.

8. Zusammensetzung nach einem der voranstehenden Ansprüche, **dadurch gekennzeichnet, dass** der Wirkstoff ein Hemikalziumsalz des Atorvastatins ist, dass ein unter Verwendung von Cu-Kₐₗₚₕₐ-Strahlung erhaltenes Pulver-Röntgenbeugungsmuster mit Signalen bei den Beugungswinkeln 2thetha von 4,12°, 4,99°, 5,77°, 7,67°, 8,45°, 15,96°, 16,62°, 17,73°, 18,27°, 18,87°, 19,48°, 19,98°, 20,29°, 21,11°, 21,67°, 23,32°, 24,41°, 24,97° und 25,40° ± 0,2° aufweist.

9. Zusammensetzung nach einem der voranstehenden Ansprüchen, **dadurch gekennzeichnet, dass** die Zusammensetzung eine Tablette oder eine Kapsel ist.

10. Zusammensetzung nach einem der voranstehenden Ansprüchen, **dadurch gekennzeichnet, dass** die Zusammensetzung frei von einem Tensid ist.

11. Zusammensetzung nach einem der voranstehenden Ansprüche, **dadurch gekennzeichnet, dass** die Zusammensetzung eine *in* vitro-Freisetzungsrate des Wirkstoffs, gemessen unter Anwendung der Blattrührer-Methode gemäß Eur. Ph. bei 75 U/min in 900 ml Phosphatpuffer mit einem pH-Wert von 6,8 bei 37 ± 0,5 °C, von 35 % bis 50 % in 5 min, von 65 % bis 90 % in 10 min, von 70 % bis 95 % in 15 min, von 80 % bis 100 % in 30 min und von 85 % bis 100 % in 45 min aufweist.

12. Zusammensetzung nach einem der voranstehenden Ansprüche, **dadurch gekennzeichnet, dass** die Zusammensetzung ein granuläres Trockenbindemittel umfasst.

13. Pulverförmige Mischung zur Herstellung einer Zusammensetzung nach einem der Ansprüche 1 bis 12, umfassend als Wirkstoff ein Salz des Atorvastatins in frei fließender kristalliner Form, wobei der Wirkstoff eine Partikelgrößenverteilung mit einem D90-Wert von kleiner als 100 *µ*m aufweist.

14. Verwendung einer pulverförmige Mischung nach Anspruch 13 zur Herstellung einer Zusammensetzung nach einem der Ansprüche 1 bis 12.

15. Verfahren zur Herstellung einer Zusammensetzung nach einem der Ansprüche 1 bis 12, umfassend den Schritt des Verpressens einer pulverförmigen Mischung nach Anspruch 13.
